# EUROPEAN PATENT APPLICATION

(11) **EP 4 523 715 A1**
(43) Date of publication of application: **19.03.2025**
(21) Application number: 23830931.4
(22) Date of filing: 29.05.2023
(51) Int. Cl.: A61M 1/16

(54) **BLOOD PURIFICATION DEVICE AND METHOD FOR CONTROLLING LIQUID FEED PUMP**

(30) Priority: 28.06.2022 JP 2022103781
(71) Applicant: Nikkiso Co., Ltd., Tokyo 150-6022 (JP)
(72) Inventor: KATAYAMA, Yuki, Makinohara-shi Shizuoka 421-0496 (JP)
(74) Representative: Grosse Schumacher Knauer von Hirschhausen
(86) International application number: PCT/JP2023/019908
(87) International publication number: WO 2024/004481

(57) **Abstract**

This blood purification device comprises: a dialysate circuit 41 that supplies a dialysate to a dialyzer 10 and discharges the dialysate from the dialyzer 10; a liquid supply pump 63 provided in the dialysate circuit 41; and a control section 22 that executes a liquid supply pump drive control operation for controlling the liquid supply pump 63 so that the flow rate of the liquid supply pump 63 is a target flow rate. In the liquid supply pump drive control operation, feedforward control is performed, and the control is switched from the feedforward control to feedback control on the condition that a predetermined period of time has elapsed.

## Description

### TECHNICAL FIELD

The present invention relates to a blood purification device and a method for controlling liquid feed pump.

### BACKGROUND OF THE INVENTION

There is a blood purification device in which drive control of a liquid feed pump is performed by feedback control (see Patent Literature 1). This blood purification device includes a blood purifier, a dialysate line to supply dialysate to the blood purifier, a liquid discharge line to discharge a waste liquid from the blood purifier, a liquid feed pump (a dialysate pump) arranged on the dialysate line, and a water balance detection unit to detect the amount of water balance between the dialysate and the waste liquid (a water removal amount), and controls drive of the liquid feed pump by feeding back a detection result detected by the water balance detection unit.

### CITATION LIST

### PATENT LITERATURES

Patent Literature 1: WO 2020/137190

### SUMMARY OF THE INVENTION

In the case of the conventional blood purification device, it takes time until the flow rate of the liquid feed pump reaches close to the target flow rate, and also, the feedback control of the liquid feed pump may cause fluctuations in the flow rate of the dialysate. Fluctuations in the flow rate, when occur, disturb feedback control of other actuators (e.g., a heater, etc.).

Therefore, it is an object of the invention to provide a blood purification device and a method for controlling liquid feed pump, which are capable of reducing time until a flow rate of a liquid feed pump reaches close to a target flow rate, and are also capable of suppressing flow rate fluctuations due to feedback control.

A blood purification device in an embodiment of the invention is a blood purification device to perform blood purification treatment through a blood purifier that purifies blood of a patient, the blood purification device comprising:
a dialysate circuit to supply dialysate to the blood purifier and discharge dialysate from the blood purifier;
a liquid feed pump arranged on the dialysate circuit to feed dialysate in the dialysate circuit;
a drive control unit that executes a drive control operation to control the liquid feed pump so that a flow rate of the liquid feed pump achieves a target flow rate; and
a flow rate detection unit to detect the flow rate of the liquid feed pump,
wherein in the drive control operation, feedforward control not based on a detection value of the flow rate detection unit is implemented, and the control is switched from the feedforward control to feedback control based on a detection value of the flow rate detection unit on the condition that a predetermined time has elapsed or a predetermined flow rate has been reached.

In addition, a method for controlling liquid feed pump in an embodiment of the invention is a method for controlling liquid feed pump to control a liquid feed pump arranged on a dialysate circuit of a blood purification device so that a flow rate of the fluid feed pump achieves a target flow rate, the method comprising:
implementing feedforward control not based on a detection value of a flow rate detection unit that detects the flow rate of the liquid feed pump, and switching from the feedforward control to feedback control based on a detection value of the flow rate detection unit on the condition that a predetermined time has elapsed or a predetermined flow rate has been reached.

### Advantageous Effects of the Invention

According to an embodiment of the invention, it is possible to reduce time until the flow rate of the liquid feed pump reaches close to the target flow rate, and also possible to suppress flow rate fluctuations due to feedback control.

### BRIEF DESCRIPTION OF DRAWINGS

Fig. 1 is a schematic structural diagram illustrating a structure of a blood purification device in an embodiment of the present invention.
Fig. 2 is a flowchart showing the first half of a calibration operation.
Fig. 3 is a flowchart showing the second half of the calibration operation.
Fig. 4 is a graph showing an example of command value and flow rate versus time in a liquid supply pump drive control operation and a liquid discharge pump drive control operation.
Fig. 5 is a flowchart showing the liquid supply pump drive control operation.
Fig. 6 is a flowchart showing the liquid discharge pump drive control operation.
Fig. 7 is a flowchart showing a modification of the liquid supply pump drive control operation.

### DETAILED DESCRIPTION OF THE INVENTION

A blood purification device in an embodiment of the invention will be described below in reference to the appended drawings. This blood purification device is a medical device that performs dialysis treatment to purify patient's blood using a dialyzer. In particular, this blood purification device uses feedforward control and feedback control so as to be able to quickly reach close to a target flow rate and to achieve stable flow rate control.

### Configuration of Blood purification device

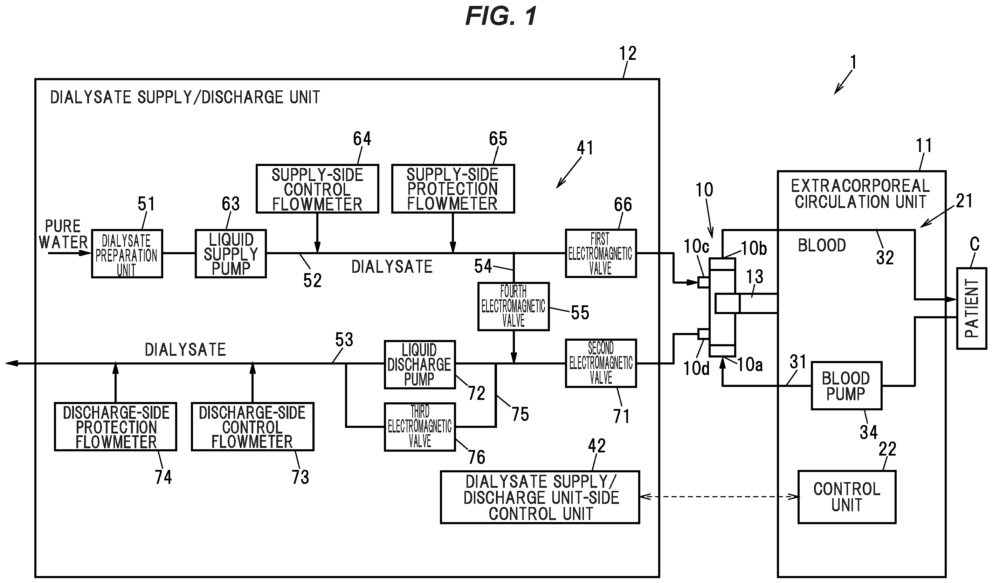

As shown in Fig. 1, a blood purification device 1 includes a dialyzer 10 that purifies blood of a patient C, an extracorporeal circulation unit 11 that circulates the blood of the patient C through the dialyzer 10, and a dialysate supply/discharge unit 12 that is connected to the dialyzer 10, supplies dialysate to the dialyzer 10 and discharges dialysate from the dialyzer 10. The extracorporeal circulation unit 11 and the dialysate supply/discharge unit 12 are configured as separate units, and the dialyzer 10 is removably attached to the extracorporeal circulation unit 11 through a fixing tool 13. The dialyzer 10 is an example of the blood purifier.

The dialyzer 10 has a blood purification membrane (a hollow-fiber hemodialysis membrane or hemodialysis filtration membrane, or a flat hemodialysis membrane or hemofiltration membrane) thereinside. The dialyzer 10 also has a blood inlet 10a to introduce blood and a blood outlet 10b to discharge the introduced blood, as well as a dialysate inlet 10c to introduce dialysate and a dialysate outlet 10d to discharge the introduced dialysate. In the dialyzer 10, blood is purified by bringing the blood into contact with dialysate through the blood purification membrane. In addition, in the dialyzer 10, removal of water from the blood is made possible by controlling liquid supply and liquid discharge in the dialysate supply/discharge unit 12.

The extracorporeal circulation unit 11 has an extracorporeal circulation circuit 21 that circulates the blood of the patient C through the dialyzer 10, and a control unit 22. The control unit 22 will be described later.

The extracorporeal circulation circuit 21 has an artery-side blood flow path 31 that is connected to the blood inlet 10a of the dialyzer 10 and leads the blood collected from a blood vessel of the patient C to the dialyzer 10, a vein-side blood flow path 32 that is connected to the blood outlet 10b of the dialyzer 10 and returns the blood discharged from the dialyzer 10 to the blood vessel of the patient C, and a blood pump 34 arranged on the artery-side blood flow path 31 to circulate the blood. The blood from the patient C is led to the dialyzer 10 through the artery-side blood flow path 31 by driving the blood pump 34, and the blood is purified by the dialyzer 10 and is then returned to the patient C through the vein-side blood flow path 32. The blood of the patient C is thereby purified.

### Configuration of Dialysate supply/discharge unit

The dialysate supply/discharge unit 12 has a dialysate circuit 41 that supplies dialysate to the dialyzer 10 and also discharges dialysate from the dialyzer 10, and a dialysate supply/discharge unit-side control unit 42.

The dialysate circuit 41 has a dialysate preparation unit 51 that refines dialysate, a dialysate supply flow path 52 that is connected to the dialysate inlet 10c of the dialyzer 10 and supplies unused dialysate refined by the dialysate preparation unit 51 to the dialyzer 10, a dialysate discharge flow path 53 that is connected to the dialysate outlet 10d of the dialyzer 10 and collects and discharges used dialysate from the dialyzer 10, and a first bypass flow path 54 that is connected to the dialysate supply flow path 52 and the dialysate discharge flow path 53 before and after the dialyzer 10 and connects the dialysate supply flow path 52 to the dialysate discharge flow path 53 without passing through the dialyzer 10.

The dialysate preparation unit 51 prepares dialysate from pure water supplied thereto and a dialysate agent made of a concentrated solution or powder. The pure water supplied to the dialysate preparation unit 51 may be supplied from a pure water production unit mounted on the dialysate supply/discharge unit 12, or may be supplied from a pure water production device provided outside the dialysate supply/discharge unit 12. In this regard, the dialysate preparation unit 51 can be omitted, and the configuration may be such that, e.g., dialysate is supplied to the dialysate supply/discharge unit 12 from an external dialysate supply device, etc.

From the upstream side, a liquid supply pump 63, a liquid supply-side control flowmeter 64, a supply-side protection flowmeter 65 and a first electromagnetic valve 66 are arranged on the dialysate supply flow path 52. The liquid supply pump 63 is a liquid feed pump that feeds dialysate in the dialysate supply flow path 52. The dialysate is supplied to the dialyzer 10 by driving the liquid supply pump 63. The liquid supply pump 63 is an example of the first liquid feed pump.

The supply-side control flowmeter 64 and the supply-side protection flowmeter 65 are flowmeters that are arranged on the downstream side of the liquid supply pump 63 and detect the flow rate of the liquid supply pump 63 (i.e., the flow rate of the dialysate). The supply-side control flowmeter 64 is a flowmeter for control that detects the flow rate of the liquid supply pump 63 to control the liquid supply pump 63. On the other hand, the supply-side protection flowmeter 65 is a flowmeter for protection to ensure that the supply-side control flowmeter 64 and its detection value are normal. The supply-side control flowmeter 64 is an example of the flow rate detection unit.

The first electromagnetic valve 66 is arranged on the downstream side of a connection position connected to the first bypass flow path 54, and is one of electromagnetic valves to perform flow path switching from the flow path passing through the dialyzer 10 to the first bypass flow path 54.

From the upstream side, a second electromagnetic valve 71, a liquid discharge pump 72, a discharge-side control flowmeter 73 and a discharge-side protection flowmeter 74 are arranged on the dialysate discharge flow path 53. A second bypass flow path 75 connected before and after the liquid discharge pump 72 is also arranged on the dialysate discharge flow path 53.

The second electromagnetic valve 71 is arranged on the upstream side of a connection position connected to the first bypass flow path 54, and is one of the electromagnetic valves to perform the flow path switching from the flow path passing through the dialyzer 10 to the first bypass flow path 54.

The liquid discharge pump 72 is a liquid feed pump that feeds dialysate in the dialysate discharge flow path 53. The dialysate from the dialyzer 10 is discharged by driving the liquid discharge pump 72. The flow rate of the liquid supply pump 63 and the flow rate of the liquid discharge pump 72 are adjusted by controlling the liquid supply pump 63 and the liquid discharge pump 72, thereby controlling the amount of water removed from the blood in the dialyzer 10. The liquid discharge pump 72 is an example of the second liquid feed pump.

The discharge-side control flowmeter 73 and the discharge-side protection flowmeter 74 are flowmeters that are arranged on the downstream side of the liquid discharge pump 72 and detect the flow rate of the liquid discharge pump 72 (i.e., the flow rate of the dialysate). The discharge-side control flowmeter 73 is a flowmeter for control that detects the flow rate of the liquid discharge pump 72 to control the liquid discharge pump 72. On the other hand, the discharge-side protection flowmeter 74 is a flowmeter for protection to ensure that the discharge-side control flowmeter 73 and its detection value are normal. The discharge-side control flowmeter 73 is an example of the flow rate detection unit.

The second bypass flow path 75 is a flow path to release the pressure in the dialysate discharge flow path 53 and is connected to the dialysate discharge flow path 53 before and after the liquid discharge pump 72. In addition, a third electromagnetic valve 76 is arranged on the second bypass flow path 75 to switch a flow path state between a connected state in which the second bypass flow path 75 is connected to the dialysate discharge flow path53 and a disconnected state in which the second bypass flow path 75 is not connected to the dialysate discharge flow path 53. By opening the third electromagnetic valve 76, the dialysate discharge flow path 53 and the second bypass flow path 75 are connected to each other, the pressure in the dialysate circuit 41 is released, and the dialysate discharge flow path 53 is placed in a pressure-released state. On the other hand, by closing the third electromagnetic valve 76, the connection between the dialysate discharge flow path 53 and the second bypass flow path 75 is cut off and the dialysate discharge flow path 53 is returned to the normal state (a state with no pressure released).

A fourth electromagnetic valve 55 is arranged on the first bypass flow path 54. The flow path is switched from the flow path passing through the dialyzer 10 to the first bypass flow path 54 by closing the first electromagnetic valve 66 and the second electromagnetic valve 71 while opening the fourth electromagnetic valve 55. On the other hand, the flow path is switched from the first bypass flow path 54 to the flow path passing through the dialyzer 10 by opening the first electromagnetic valve 66 and the second electromagnetic valve 71 while closing the fourth electromagnetic valve 55.

The dialysate supply/discharge unit-side control unit 42 communicates with the control unit 22 of the extracorporeal circulation unit 11, and controls the liquid supply pump 63, the liquid discharge pump 72 and each of the electromagnetic valves 55, 66, 71 and 76 according to commands from the control unit 22. The dialysate supply/discharge unit-side control unit 42 is realized by appropriately combining an arithmetic element such as CPU, a memory, software, interface and a communication unit, etc.

### Description of Control unit and its control

The control unit 22 of the extracorporeal circulation unit 11 and the control by this control unit 22 will now be described in reference to Figs. 1 to 6. The control unit 22 controls the blood pump 34, and also communicates with the dialysate supply/discharge unit-side control unit 42 and controls the liquid supply pump 63, the liquid discharge pump 72 and each of the electromagnetic valves 55, 66, 71, and 76 through the dialysate supply/discharge unit-side control unit 42. The control unit 22 also receives a detection value of each of the flowmeters 64, 65, 73 and 74 through the dialysate supply/discharge unit-side control unit 42. In particular, the control unit 22 executes a calibration operation, a liquid supply pump drive control operation, and a liquid discharge pump drive control operation. The control unit 22 is realized by appropriately combining an arithmetic element such as CPU, a memory, software, interface and a communication unit, etc. The control unit 22 is an example of the drive control unit and the calibration unit, and the liquid supply pump drive control operation and the liquid discharge pump drive control operation are examples of the drive control operation and a method for controlling drive of liquid feed pump.

The calibration operation is an operation to obtain an expression of the proportional relationship between the command value and the flow rate to be used for feedforward control, and involves obtaining command values at two flow rates different from each other and, based on these, calculating the expression of the proportional relationship. The expression of the proportional relationship is an example of the calibration information associating the command values of the pumps 63 and 72 with the flow rates. In the present embodiment, 300 mL/min and 500 mL/min are used as an example of the above-mentioned two flow rates.

In the present embodiment, for example, a DA value is used as the command value. This DA value is a command value that is output from the control unit 22 to each pump (the liquid supply pump 63, the liquid discharge pump 72, etc.) through the dialysate supply/discharge unit-side control unit 42. To be precise, the DA value is output as a digital signal of 16-bit expression from a microcomputer of the control unit 22 to a DA converter through a microcomputer of the dialysate supply/discharge unit-side control unit 42 and is converted into a corresponding command voltage, and the converted command voltage is input to each of the pumps 63 and 72. Each of the pumps 63 and 72 is driven by the input command voltage. In this regard, the configuration may be such that the control unit 22 or the dialysate supply/discharge unit-side control unit 42 has a DA converter and converts the DA value into the command voltage, or that the blood purification device 1 has a DA converter as a separate unit from the control unit 22 and the dialysate supply/discharge unit-side control unit 42.

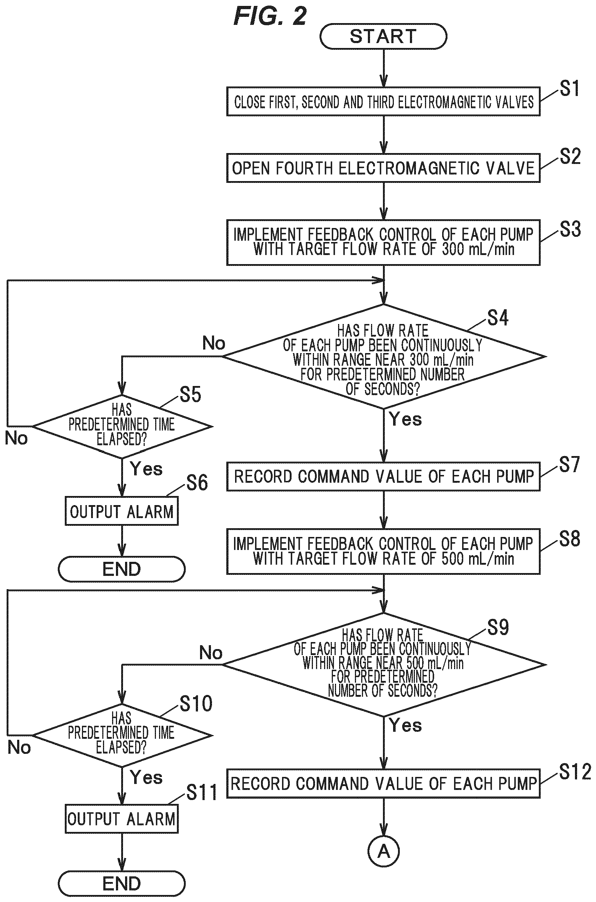

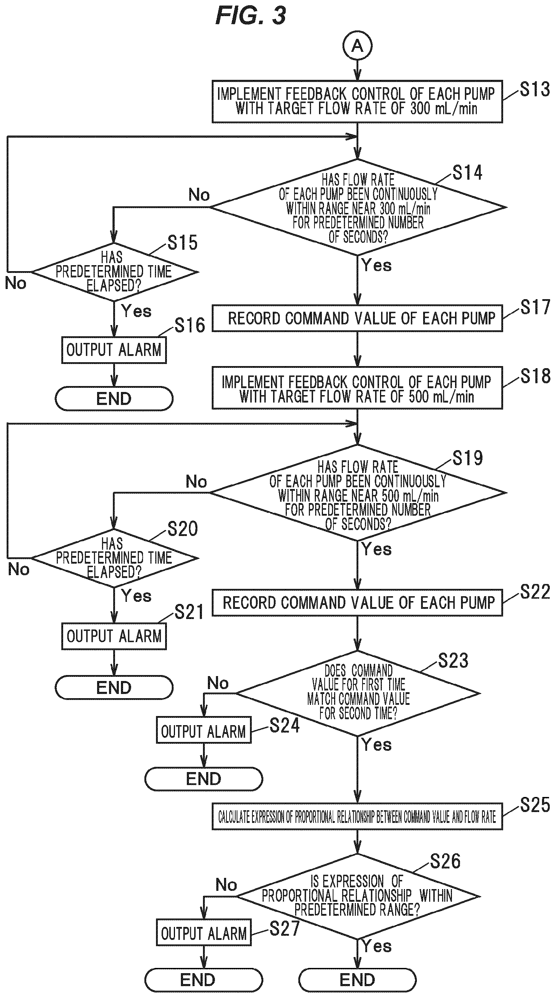

Figs. 2 and 3 are flowcharts showing the calibration operation. In the calibration operation, first, the first electromagnetic valve 66, the second electromagnetic valve 71 and the third electromagnetic valve 76 are closed (S1), and the fourth electromagnetic valve 55 is opened (S2), as shown in Fig. 2. This switches the flow path from the flow path passing through the dialyzer 10 to the first bypass flow path 54 and also places the dialysate discharge flow path 53 into a state with no pressure released.

After that, the liquid supply pump 63 and the liquid discharge pump 72 are driven by feedback control with a target flow rate of 300 mL/min (S3). This creates a state in which dialysate is fed at a flow rate of 300 mL/min. When the flow rate detected by each of the control flowmeters 64 and 73 continuously fails to fall within a range near 300 mL/min (e.g., within a range of ±2) for a predetermined number of seconds under the feedback control (S4: No) and a predetermined time has elapsed (S5: Yes), it is determined that there is an abnormality in each of the pumps 63, 72 or each of the control flowmeters 64, 73, an alarm is output (S6), and this operation ends. In other words, when the flow rates of the liquid supply pump 63 and the liquid discharge pump 72 do not reach the target flow rates by feedback control, it is determined that there is an abnormality in each of the pumps 63, 72 or each of the control flowmeters 64, 73. In this regard, the feedback control is a control in which, while the detection values (flow rates) of the control flowmeters 64, 73 are periodically fed back to change the command values, the pumps 63 and 72 are driven according to the changed command values.

On the other hand, when the flow rate detected by each of the control flowmeters 64 and 73 is continuously within the range near 300 mL/min for a predetermined number of seconds under the feedback control (S4: Yes), the command values of the liquid supply pump 63 and the liquid discharge pump 72 are recorded (S7). The command value of each of the pumps 63 and 72 at a flow rate of 300 mL/min is thereby obtained.

After that, the liquid supply pump 63 and the liquid discharge pump 72 are driven by feedback control with a target flow rate of 500 mL/min (S8). This creates a state in which the dialysate is fed at a flow rate of 500 mL/min. When the flow rate detected by each of the control flowmeters 64 and 73 continuously fails to fall within a range near 500 mL/min (e.g., within a range of ±2) for a predetermined number of seconds under the feedback control (S9: No) and a predetermined time has elapsed (S10: Yes), it is determined that there is an abnormality in each of the pumps 63, 72 or each of the control flowmeters 64, 73, an alarm is output (S11), and this operation ends.

On the other hand, when the flow rate detected by each of the control flowmeters 64 and 73 is continuously within the range near 500 mL/min for a predetermined number of seconds under the feedback control (S9: Yes), the command values of the liquid supply pump 63 and the liquid discharge pump 72 are recorded (S12). The command value of each of the pumps 63 and 72 at a flow rate of 500 mL/min is thereby obtained.

After finishing the process of recording the command values for the first time (S2 to S12), the process of recording the command values for the second time (S13 to S22) for confirmation is performed as shown in Fig. 3. That is, similarly to the recording process for the first time, first, the liquid supply pump 63 and the liquid discharge pump 72 are driven by feedback control with a target flow rate of 300 mL/min (S13). When the flow rate detected by each of the control flowmeters 64 and 73 continuously fails to fall within a range near 300 mL/min for a predetermined number of seconds under the feedback control (S14: No) and a predetermined time has elapsed (S15: Yes), it is determined that there is an abnormality in each of the pumps 63, 72 or each of the control flowmeters 64, 73, an alarm is output (S16), and this operation ends.

On the other hand, when the flow rate detected by each of the control flowmeters 64 and 73 is continuously within the range near 300 mL/min for a predetermined number of seconds under the feedback control (S14: Yes), the command values of the liquid supply pump 63 and the liquid discharge pump 72 are recorded (S17).

After that, the liquid supply pump 63 and the liquid discharge pump 72 are driven by feedback control with a target flow rate of 500 mL/min (S18). When the flow rate detected by each of the control flowmeters 64 and 73 continuously fails to fall within a range near 500 mL/min for a predetermined number of seconds under the feedback control (S19: No) and a predetermined time has elapsed (S20: Yes), it is determined that there is an abnormality in each of the pumps 63, 72 or each of the control flowmeters 64, 73, an alarm is output (S21), and this operation ends. On the other hand, when the flow rate detected by each of the control flowmeters 64 and 73 is continuously within the range near 500 mL/min for a predetermined number of seconds under the feedback control (S18: Yes), the command values of the liquid supply pump 63 and the liquid discharge pump 72 are recorded (S22).

After finishing the two recording processes, whether or not the command value recorded for the first time matches the command value recorded for the second time is determined for each flow rate (300 mL/min and 500 mL/min) of each of the pumps 63 and 72 (S23). When it is determined that the command value for the first time does not match the command value for the second time (S23: No), an alarm is output (S24) and this operation ends. On the other hand, when it is determined that the command values for the first time match the command values for the second time (S23: Yes), the expression of the proportional relationship between the command value and the flow rate is calculated based on the command values for the second time (S25). That is, the expression of the proportional relationship between the command value and the flow rate for the liquid supply pump 63 is calculated based on the command value of the liquid supply pump 63 when the flow rate is 300 mL/min and the command value of the liquid supply pump 63 when the flow rate is 500 mL/min, which are recorded in the recording process for the second time. Likewise, the expression of the proportional relationship between the command value and the flow rate for the liquid discharge pump 72 is calculated based on the command value of the liquid discharge pump 72 when the flow rate is 300 mL/min and the command value of the liquid discharge pump 72 when the flow rate is 500 mL/min, which are recorded in the recording process for the second time.

After calculating the expression of the proportional relationship for each of the pumps 63 and 72, whether or not there is an abnormality in each pump 63, 72 or each control flowmeter 64, 72 is determined based on whether or not (mainly the slope of) the expression of the proportional relationship for each of the pumps 63 and 72 is within a predetermined range. That is, whether or not there is an abnormality in each of the pumps 63, 72 or each of the control flowmeters 64, 73 is determined based on whether the calculated expression of the proportional relationship for each of the pumps 63 and 72 is true or false. When the expression of the proportional relationship for each of the pumps 63 and 72 is beyond the predetermined range and it is determined to be an abnormal value (S26: No), it is determined that there is an abnormality in each of the pumps 63, 72 or each of the control flowmeters 64, 73, and an alarm is output (S27). On the other hand, when the expression of the proportional relationship for each of the pumps 63 and 72 is within the predetermined range and it is determined to be a normal value (S26: Yes), it is determined that there is no abnormality in each of the pumps 63, 72 or each of the control flowmeters 64, 73, and this operation ends. In this manner, in the present embodiment, by performing an abnormality determination for each of the pumps 63, 72 or each of the control flow meters 64, 73 during the calibration operation to obtain the expression of the proportional relationship, it is possible to easily detect an abnormality.

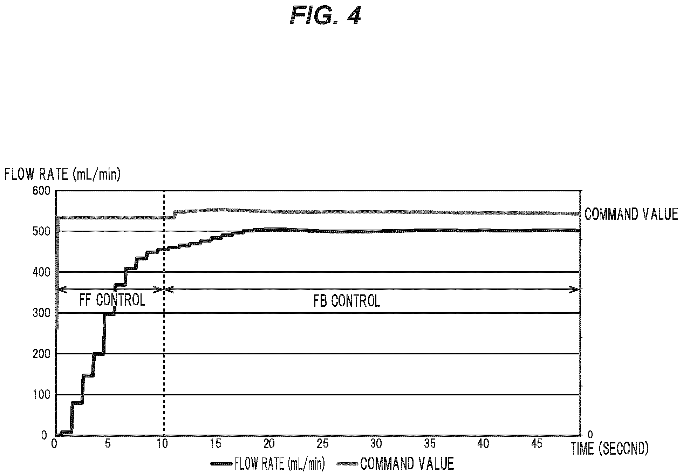

The liquid supply pump drive control operation is an operation to control the drive of the liquid supply pump 63 so that the flow rate of the liquid supply pump 63 achieves the target flow rate, and as shown in Fig. 4, feedforward control (FF control) not based on the detection value of the supply-side control flowmeter 64 is performed until a predetermined time estimated to reach the target flow rate by the feedforward control has elapsed, and after this predetermined time has elapsed, the control is switched from the feedforward control to feedback control (FB control) based on the detection value of the supply-side control flowmeter 64. The feedforward control is a control where the pumps 63 and 72 are driven by a certain command value, without being based on the detection value of the supply-side control flowmeter 64. In the liquid supply pump drive control operation, a command value corresponding to the target flow rate, which is calculated based on the expression of the proportional relationship obtained in the calibration operation, is used as a command value in the feedforward control. It is estimated that when driving the liquid supply pump 63 for 10 seconds under the feedforward control according to this command value, the flow rate of the liquid supply pump 63 reaches the target flow rate, hence, the above-mentioned "predetermined time estimated to reach the target flow rate by the feedforward control" is set to 10 seconds in the present embodiment.

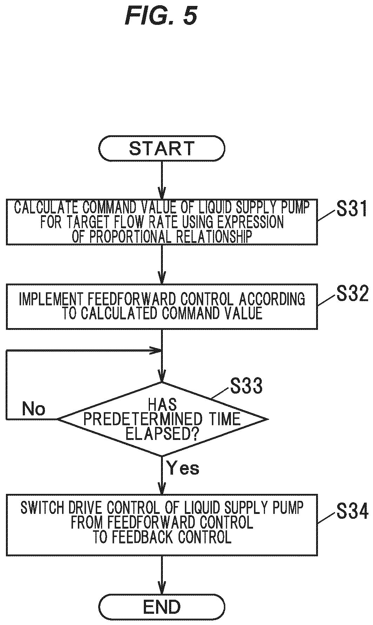

Fig. 5 is a flowchart showing the liquid supply pump drive control operation. In the liquid supply pump drive control operation, first, as shown in Fig. 5, the command value corresponding to the target flow rate is calculated based on the expression of the proportional relationship for the liquid supply pump 63 obtained in the calibration operation (S31). After that, the liquid supply pump 63 is driven by the feedforward control according to the calculated command value (S32). After the above-mentioned predetermined time (10 seconds) has elapsed since starting to drive the liquid supply pump 63 under the feedforward control (S33: Yes), the drive control of the liquid supply pump 63 is switched from the feedforward control to the feedback control (S34). This operation thereby ends.

The liquid discharge pump drive control operation is an operation to control the drive of the liquid discharge pump 72 so that the flow rate of the liquid discharge pump 72 achieves the target flow rate, and as shown in Fig. 4, feedforward control not based on the detection value of the discharge-side control flowmeter 73 is performed until a predetermined time estimated to reach the target flow rate by the feedforward control has elapsed, and after this predetermined time has elapsed, the control is switched from the feedforward control to feedback control based on the detection value of the discharge-side control flowmeter 73. In the liquid discharge pump drive control operation, a command value corresponding to the target flow rate, which is calculated based on the expression of the proportional relationship obtained in the calibration operation, is used as a command value in the feedforward control. It is estimated that when driving the liquid discharge pump 72 for 10 seconds under the feedforward control according to this command value, the flow rate of the liquid discharge pump 72 reaches the target flow rate, hence, the above-mentioned "predetermined time estimated to reach the target flow rate by the feedforward control" is set to 10 seconds in the present embodiment.

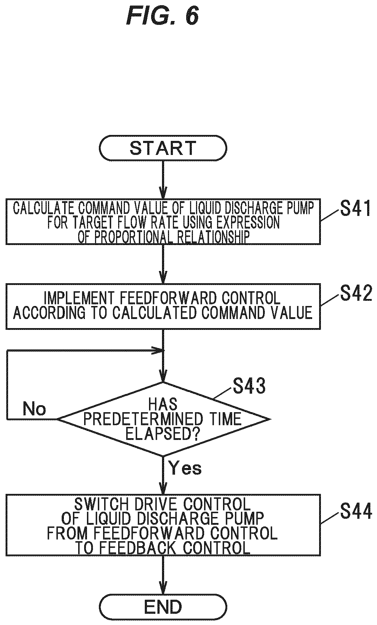

Fig. 6 is a flowchart showing the liquid discharge pump drive control operation. In the liquid discharge pump drive control operation, first, as shown in Fig. 6, the command value corresponding to the target flow rate is calculated based on the expression of the proportional relationship for the liquid discharge pump 72 obtained in the calibration operation (S41). After that, the liquid discharge pump 72 is driven by the feedforward control according to the calculated command value (S42). After the above-mentioned predetermined time (10 seconds) has elapsed since starting to drive the liquid discharge pump 72 under the feedforward control (S43: Yes), the drive control of the liquid discharge pump 72 is switched from the feedforward control to the feedback control (S44). This operation thereby ends.

The blood purification device 1 executes a startup step, a treatment preparation step, a step of inserting needle and removing blood from patient, a treatment step, a treatment suspension step, a step of removing needle and return blood to patient, a liquid drainage step, a cleaning and disinfection step, a standby step, and an adjustment step. The calibration operation is executed during self-diagnosis in the startup step, and during the adjustment step.

Meanwhile, the liquid supply pump drive control operation is executed at the time of starting up the liquid supply pump 63 in the startup step, at the time of restarting the liquid supply pump 63 in the treatment suspension step, and at the time of changing the target flow rate of the liquid supply pump 63 in the preparation step, in the step of inserting needle and removing blood from patient, in the treatment step, and in the step of removing needle and return blood to patient. That is, the control unit 22 executes the above-mentioned liquid supply pump drive control operation as the control operation of the liquid supply pump 63 at the time of starting up the liquid supply pump 63 (when the liquid supply pump 63 is started), at the time of restarting the liquid supply pump 63 (when the liquid supply pump 63 is restarted), and at the time of changing the target flow rate of the liquid supply pump 63 (when the target flow rate of the liquid supply pump 63 is changed). **In** this way, by configuring such that the liquid supply pump drive control operation, in which the feedforward control is first performed and the control is then switched to the feedback control, is performed at the time of starting up or restarting the liquid supply pump 63 or changing the target flow rate, it is possible to reduce the time until the flow rate of the liquid supply pump 63 reaches close to the target flow rate, and it is also possible to suppress flow rate fluctuations due to feedback control.

Then, the liquid discharge pump drive control operation is executed when the third electromagnetic valve 76 in the open state is closed in the startup step, the treatment preparation step, the step of inserting needle and removing blood from patient, the treatment step, the treatment suspension step, the step of removing needle and return blood to patient, the cleaning and disinfection step, the standby step, and the adjustment step. That is, the liquid discharge pump drive control operation is executed when changing from the state in which the dialysate discharge flow path 53 and the second bypass flow path 75 are connected by opening the third electromagnetic valve 76, to the state in which the dialysate discharge flow path 53 and the second bypass flow path 75 are disconnected by closing the third electromagnetic valve 76. In other words, the control unit 22 executes the above-mentioned liquid discharge pump drive control operation as the control operation of the liquid discharge pump 72 when switching the second bypass flow path 75 from the connected state to the disconnected state by the third electromagnetic valve 76 (when switching from the connected state to the disconnected state by the third electromagnetic valve 76). In this way, by configuring such that the liquid discharge pump drive control operation, in which the feedforward control is first performed and the control is then switched to the feedback control, is performed at the time of switching the connection of the second bypass flow path 75, it is possible to drive the liquid discharge pump 72 without being affected by detection error of the discharge-side control flowmeter 73 which occurs when switching the connection of the second bypass flow path 75. The configuration may be such that the feedforward control is implemented during when the third electromagnetic valve 76 is open, and the control is switched from the feedforward control to the feedback control when the third electromagnetic valve 76 is closed.

### Functions and Effects of the embodiment

In the configuration of the embodiment described above, by performing the drive control operation in which the feedforward control is first implemented and the control is then switched from the feedforward control to the feedback control, it is possible to reduce the time until the flow rates of the pumps 63 and 72 reach close to the target flow rates as shown in Fig. 4. In addition, since the feedback control is started in the state in which the flow rates of the pumps 63 and 72 have reached close to the target flow rates, it is possible to suppress flow rate fluctuations due to feedback control. Particularly in the configuration in which two pumps 63, 72 are arranged on the dialysate circuit 41 as in the present embodiment, the feedback control of the pumps 63, 72 is affected by each other, hence, a disturbance with large amplitude is likely to occur. In addition, in the present embodiment, since temperature adjustment of dialysate by a temperature adjustment unit (not shown) or concentration adjustment in the dialysate preparation unit 51 is performed simultaneously with the flow rate adjustment by the pumps 63 and 72, feedback controls for these adjustments affect each other and a disturbance with large amplitude is likely to occur. However, the drive control operation described above can prevent or suppress occurrence of a disturbance with large amplitude. Since this can suppress pressure fluctuations in the dialysate circuit 41, it is possible to suppress not only the load on the tube of the dialysate circuit 41 but also the load on the dialyzer 10 and the load on the patient C.

In addition, in the configuration of the embodiment described above, by configuring such that the liquid discharge pump drive control operation, in which the feedforward control is first performed and the control is then switched to the feedback control, is performed at the time of switching the connection of the second bypass flow path 75, it is not affected by detection error of the discharge-side control flowmeter 73 which occurs when switching the connection of the second bypass flow path 75. As a result, stable flow rate control can be performed also when switching the connection of the second bypass flow path 75.

In addition, in the configuration of the embodiment described above, since the expression of the proportional relationship between the command value and the flow rate for the pumps 63, 72 is obtained by performing the calibration operation and the command value based on this is used to implement the feedforward control, it is possible to implement the feedforward control with high accuracy.

### Modifications

Although the embodiment of the invention has been described, the invention according to claims is not to be limited to the embodiment described above. Further, please note that not all combinations of the features described in the embodiment are necessary to solve the problem of the invention.

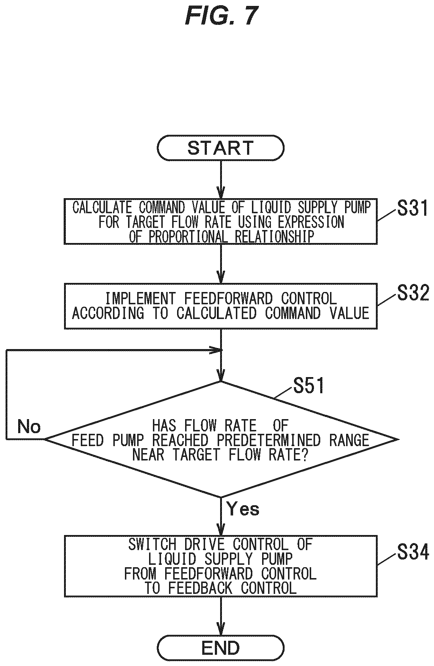

For example, the configuration in the embodiment described above is such that the control in the liquid supply pump drive control operation and the liquid discharge pump drive control operation is switched from the feedforward control to the feedback control on the condition that a predetermined time has elapsed. However, it may be configured to switch from the feedforward control to the feedback control on the condition that a predetermined flow rate is reached. In particular, the configuration may be such that, e.g., on the condition that the flow rates of the pumps 63 and 72 reach ranges near the target flow rates, the control is switched from the feedforward control to the feedback control at the time the flow rates of the pumps 63 and 72 reach the ranges near the target flow rates. That is, the configuration may be such that in the liquid supply pump drive control operation and the liquid discharge pump drive control operation, the control unit 22 implements the feedforward control until the flow rate reaches a predetermined range near the target flow rate, and switches from the feedforward control to the feedback control after the flow rate reaches the predetermined range near the target flow rate. In such a case, for example, a step of monitoring the flow rate of the liquid supply pump 63 by a flow rate detection unit such as the supply-side control flowmeter 64 and determining whether or not the flow rate of the liquid supply 63 has reached the predetermined range near the target flow rate (e.g., within a range of ±10% of the target flow rate) (S51) is performed in place of the step of determining whether a predetermined time has elapsed (S33), as shown in Fig. 7. Then, when it is determined (detected) that the flow rate of the liquid supply pump 63 has reached the predetermined range near the target flow rate, the control is switched from the feedforward control to the feedback control. In this regard, Fig. 7 shows an example of the liquid supply pump drive control operation, and in the case of the liquid discharge pump drive control operation, the step S43 in Fig. 6 is changed to a step of monitoring the flow rate of the liquid discharge pump 72 by a flow rate detection unit such as the discharge-side control flowmeter 73 and determining whether or not the flow rate of the liquid discharge pump 72 has reached the predetermined range near the target flow rate (e.g., within a range of ±10% of the target flow rate). Alternatively, it may be configured to monitor both time and flow rate and switch from the feedforward control to the feedback control when one of the time condition and the flow rate condition is satisfied.

In addition, the configuration of the embodiment described above is such that the expression of the proportional relationship is calculated in the calibration operation based on the command values corresponding to two flow rates: the command value corresponding to a flow rate of 300 mL/min and the command value corresponding to a flow rate of 500 mL/min. However, it is not limited thereto as long as it is configured to obtain command values corresponding to plural flow rates different from each other and calculate the expression of the proportional relationship based on the obtained command values corresponding to the plural flow rates. In other words, it may be configured to obtain command values corresponding to not less than three flow rates and, based on these, calculate the expression of the proportional relationship.

In addition, the numerical values described in the above embodiment and the modifications are merely examples and may be changed as appropriate.

In addition, the feedforward control and the feedback control may be arbitrarily combined without departing from the gist of the invention. For example, the feedback control may be performed first, then switched to the feedforward control, and then switched back to the feedback control again. In addition, the control may also be switched from the feedforward control to the feedback control after temporarily suspending the control operation.

### Summary of the embodiment

Technical ideas understood from the embodiment will be described below citing the reference signs, etc., used for the embodiment. However, each reference sign, etc., described below is not intended to limit the constituent elements in the claims to the members, etc., specifically described in the embodiment.
(1) A blood purification device 1 to perform blood purification treatment through a blood purifier 10 that purifies blood of a patient C, the blood purification device comprising: a dialysate circuit 41 to supply dialysate to the blood purifier 10 and discharge dialysate from the blood purifier 10; a liquid feed pump 63, 72 arranged on the dialysate circuit 41 to feed dialysate in the dialysate circuit 41; a drive control unit 22 that executes a drive control operation to control the liquid feed pump 63, 72 so that a flow rate of the liquid feed pump 63 achieves a target flow rate; and a flow rate detection unit 64, 73 to detect the flow rate of the liquid feed pump 63, 72, wherein in the drive control operation, feedforward control not based on a detection value of the flow rate detection unit 64, 73 is implemented, and the control is switched from the feedforward control to feedback control based on a detection value of the flow rate detection unit 64, 73 on the condition that a predetermined time has elapsed or a predetermined flow rate has been reached.
   It is thereby possible to reduce the time until the flow rate of the liquid feed pump reaches close to the target flow rate and also suppress fluctuations in the flow rate of the liquid feed pump.
(2) The blood purification device 1 as defined by (1), wherein the dialysate circuit 41 comprises a dialysate supply flow path 52 to supply unused dialysate to the blood purifier 10, and a dialysate discharge flow path 53 to discharge used dialysate from the blood purifier 10, wherein a first liquid feed pump 63 arranged on the dialysate supply flow path 52 and a second liquid feed pump 72 arranged on the dialysate discharge flow path 53 are provided as the liquid feed pump 63, 72, and wherein the drive control unit 22 executes the drive control operation as a control operation of the first liquid feed pump 63 when starting up the first liquid feed pump 63, when restarting the first liquid feed pump 63, and/or when changing the target flow rate of the first liquid feed pump 63.
   It is thereby possible to reduce the time until the flow rate of the liquid feed pump reaches close to the target flow rate at the time of starting up, restarting the liquid feed pump and changing the target flow rate, and also possible to suppress fluctuations in the flow rate of the liquid feed pump.
(3) The blood purification device 1 as defined by (1), wherein the dialysate circuit 41 comprises a dialysate supply flow path 52 to supply unused dialysate to the blood purifier 10, a dialysate discharge flow path 53 to discharge used dialysate from the blood purifier 10, a bypass flow path 75 that is connected to the dialysate discharge flow path 53 and releases pressure in the dialysate discharge flow path 53, and an electromagnetic valve 76 to switch a flow path state between a connected state in which the bypass flow path 75 is connected to the dialysate discharge flow path 53 and a disconnected state in which the bypass flow path 75 is not connected to the dialysate discharge flow path 53, wherein a first liquid feed pump 63 arranged on the dialysate supply flow path 52 and a second liquid feed pump 72 arranged on the dialysate discharge flow path 53 are provided as the liquid feed pump 63, 72, and wherein the drive control unit 22 executes the drive control operation as a control operation of the second liquid feed pump 72 when switching from the connected state to the disconnected state by means of the electromagnetic valve 76.
   It is thereby possible to control the liquid feed pump without being affected by detection error of the flowmeter which occurs when switching the connection of the bypass flow path.
(4) The blood purification device 1 as defined by any one of (1) to (3), further comprising: a calibration unit 22 that obtains calibration information associating a command value of the liquid feed pump 63, 72 with the flow rate, wherein in the feedforward control, the liquid feed pump 63, 72 is controlled according to the command value based on the calibration information obtained by the calibration unit 22. It is thereby possible to implement the feedforward control with high accuracy.
(5) The blood purification device 1 as defined by (4), wherein the calibration unit 22 implements the feedback control on the liquid feed pump 63, 72 using a plurality of flow rates different from each other as target flow rates to create a state in which liquid is fed at the plurality of flow rates, thereby obtains command values of the liquid feed pump 63, 72 corresponding to the plurality of flow rates, and calculates a relational expression as the calibration information based on the obtained command values corresponding to the plurality of flow rates.
   It is thereby possible to implement the feedforward control with higher accuracy.
(6) The blood purification device 1 defined by (5), wherein the calibration unit 22 determines whether or not there is an abnormality in the liquid feed pump 63, 72 or the flow rate detection unit 64, 73, based on whether the calculated relational expression is true or false.
   Since abnormality determination can be performed during the calibration operation, it is possible to easily detect the abnormality.
(7) The blood purification device 1 as defined by (5) or (6), wherein when the flow rate of the liquid supply pump 63, 72 fails to reach the target flow rate by the feedback control during when obtaining the command values corresponding to the plurality of flow rates, the calibration unit 22 determines that there is an abnormality in the liquid feed pump 63, 72 or the flow rate detection unit 64, 73.
   Since abnormality determination can be performed during the calibration operation, it is possible to easily detect the abnormality.
(8) A method for controlling liquid feed pump 63, 72 to control a liquid feed pump 63, 72 arranged on a dialysate circuit 41 of a blood purification device 1 so that a flow rate of the fluid feed pump 63, 72 achieves a target flow rate, the method for controlling liquid feed pump 63, 72 comprising: implementing feedforward control not based on a detection value of a flow rate detection unit 64, 73 that detects the flow rate of the liquid feed pump 63, 72, and switching from the feedforward control to feedback control based on a detection value of the flow rate detection unit 64, 73 on the condition that a predetermined time has elapsed or a predetermined flow rate has been reached.
   It is thereby possible to reduce the time until the flow rate of the liquid feed pump reaches close to the target flow rate and also suppress fluctuations in the flow rate of the liquid feed pump.

### REFERENCE SIGNS LIST

1: blood purification device, 10: dialyzer, 22: control unit, 41: dialysate circuit, 52: dialysate supply flow path, 53: dialysate discharge flow path, 63: liquid supply pump, 64: supply-side control flowmeter, 72: liquid discharge pump, 73: discharge-side control flowmeter, 75: second bypass flow path, 76: third electromagnetic valve, C: patient

## Claims

1. A blood purification device to perform blood purification treatment through a blood purifier that purifies blood of a patient, the blood purification device comprising:
a dialysate circuit to supply dialysate to the blood purifier and discharge dialysate from the blood purifier;
a liquid feed pump arranged on the dialysate circuit to feed dialysate in the dialysate circuit;
a drive control unit that executes a drive control operation to control the liquid feed pump so that a flow rate of the liquid feed pump achieves a target flow rate; and
a flow rate detection unit to detect the flow rate of the liquid feed pump,
wherein in the drive control operation, feedforward control not based on a detection value of the flow rate detection unit is implemented, and the control is switched from the feedforward control to feedback control based on a detection value of the flow rate detection unit on the condition that a predetermined time has elapsed or a predetermined flow rate has been reached.

2. The blood purification device according to claim 1, wherein the dialysate circuit comprises a dialysate supply flow path to supply unused dialysate to the blood purifier, and a dialysate discharge flow path to discharge used dialysate from the blood purifier, wherein a first liquid feed pump arranged on the dialysate supply flow path and a second liquid feed pump arranged on the dialysate discharge flow path are provided as the liquid feed pump, and wherein the drive control unit executes the drive control operation as a control operation of the first liquid feed pump when starting up the first liquid feed pump, when restarting the first liquid feed pump, and/or when changing the target flow rate of the first liquid feed pump.

3. The blood purification device according to claim 1, wherein the dialysate circuit comprises a dialysate supply flow path to supply unused dialysate to the blood purifier, a dialysate discharge flow path to discharge used dialysate from the blood purifier, a bypass flow path that is connected to the dialysate discharge flow path and releases pressure in the dialysate discharge flow path, and an electromagnetic valve to switch a flow path state between a connected state in which the bypass flow path is connected to the dialysate discharge flow path and a disconnected state in which the bypass flow path is not connected to the dialysate discharge flow path, wherein a first liquid feed pump arranged on the dialysate supply flow path and a second liquid feed pump arranged on the dialysate discharge flow path are provided as the liquid feed pump, and wherein the drive control unit executes the drive control operation as a control operation of the second liquid feed pump when switching from the connected state to the disconnected state by means of the electromagnetic valve.

4. The blood purification device according to any one of claims 1 to 3, further comprising:
a calibration unit that obtains calibration information associating a command value of the liquid feed pump with the flow rate,
wherein in the feedforward control, the liquid feed pump is controlled according to the command value based on the calibration information obtained by the calibration unit.

5. The blood purification device according to claim 4, wherein the calibration unit implements the feedback control on the liquid feed pump using a plurality of flow rates different from each other as target flow rates to create a state in which liquid is fed at the plurality of flow rates, thereby obtains command values of the liquid feed pump corresponding to the plurality of flow rates, and calculates a relational expression as the calibration information based on the obtained command values corresponding to the plurality of flow rates.

6. The blood purification device according to claim 5, wherein the calibration unit determines whether or not there is an abnormality in the liquid feed pump or the flow rate detection unit, based on whether the calculated relational expression is true or false.

7. The blood purification device according to claim 5, wherein when the flow rate of the liquid supply pump fails to reach the target flow rate by the feedback control during when obtaining the command values corresponding to the plurality of flow rates, the calibration unit determines that there is an abnormality in the liquid feed pump or the flow rate detection unit.

8. A method for controlling liquid feed pump to control a liquid feed pump arranged on a dialysate circuit of a blood purification device so that a flow rate of the fluid feed pump achieves a target flow rate, the method for controlling liquid feed pump comprising:
implementing feedforward control not based on a detection value of a flow rate detection unit that detects the flow rate of the liquid feed pump, and switching from the feedforward control to feedback control based on a detection value of the flow rate detection unit on the condition that a predetermined time has elapsed or a predetermined flow rate has been reached.
